Europäisches Patentamt

European Patent Office

Office européen des brevets

(11) Publication number: **0 257 837**
**A1**

## EUROPEAN PATENT APPLICATION

(21) Application number: 87306833.2

(22) Date of filing: 31.07.87

(51) Int. Cl.⁴: **C12N 15/00** , A61K 39/108 , A61K 39/112 , A61K 39/106

(30) Priority: 19.08.86 AU 7545/86

(43) Date of publication of application:
02.03.88 Bulletin 88/09

(84) Designated Contracting States:
AT BE CH DE ES FR GB GR IT LI LU NL SE

(71) Applicant: ENTEROVAX RESEARCH PTY. LTD.
University of Adelaide
North Terrace Adelaide South Australia(AU)

(72) Inventor: Morona,Renato
10 Talbot Road
Waterloo Corner,South Australia(AU)

(74) Representative: Harding, Richard Patrick et al
Arthur R. Davies & Co. 27 Imperial Square
Cheltenham GL50 1RQ(GB)

(54) Hybrid bacterial strain.

(57) An avirulent strain of <u>Salmonella</u> including a fragment of DNA containing genes encoding the synthesis of at least a portion of the core region of an <u>E. coli</u> strain.
The avirulent strain may form the basis of a live oral vaccine against cholera.

EP 0 257 837 A1

## "Hybrid Bacterial Strain"

The present invention relates to hybrid bacterial strains which may be used for expression of high levels of Vibrio cholerae O-somatic antigen (Vc OAg)/

The genes encoding the O-antigen from both serotypes of Vibrio cholerae have been cloned into E.coli K-l2. Such plasmid clones are pPMI00l, pPMI002, pPMI003, and pPMI004 which is derived from pPMI00l. [These plasmids have been described in a previous patent application PCT/AU85/000l5, the entire disclosure of which is incorporated herein by reference, and Manning et al, (l986) Infect. Immun 53(2) 272-277]. E.coliK-l2 strains harbouring these plasmids produce high levels of Vc OAg which is in the form of a lipopolysaccharide (LPS) since it is extractable by the standard hot phenol/water method, is detectable by silver staining of cell envelope material electrophoresed on a SDS-polyacrylamide gel, and it is on the cell surface of clones as judged by agglutination with antiserum to Vibrio cholerae.

However, when pPMI004 is introduced into either Salmonella typhimurium strain G30, or into Salmonella typhi strain Ty2la, both galE mutant strains, the Vc OAg is produced at low levels and is not in the form of a lipopolysaccharide since it cannot be extracted by the hot phenol/water method and cannot be silver stained as described above. Since these, and other Salmonella strains, express the Vc OAg poorly, then this is a major obstacle to the use of Salmonellae as a carrier strain for Vc OAg in a live oral vaccine; Vc OAg is known to be a protective antigen for immunity to cholera.

Poor expression of Vc OAg in Salmonella relative to the expression obtained in E.coli K-l2 may be the result of any one of the many differences between the two species. The block (or blocks) in expression may occur at the level of gene expression or biosynthesis and assembly.

It is accordingly an object of the present invention to overcome, or at least alleviate one or more of the difficulties related to the prior art.

In accordance therewith one major difference between E.coli and Salmonella which has now been investigated and which is thought to be significant resides in the difference in the composition of the core region of the LPS between the two species. Figure I shows the arrangement and composition of the core sugars in the LPS on E.coli K-l2 and Salmonella typhimurium. It is interesting to note that a galE mutant of E. coli K-l2 would have di-glucoses as the terminal sugars in its LPS, whereas a Salmonella galE mutant would have a mono-glucose residue as the terminal sugar.

The role of the LPS core in the Vc OAg expression in E.coliK-l2 was investigated by examining the expression of Vc OAg (Ogawa type) in various E.coli K-l2 strains which have defects affecting the LPS core. In summary, E.coli K-l2 strains with a wild type or galE-type LPS core expressed Vc OAg. No expression of Vc OAg was observed in E.coli K-l2 mutant strains which lack heptose and glucose or glucose only, or have only one glucose residue in the LPS core, (Figure I). This result indicates that in E.coli K-l2 the Vc OAg is assembled onto the E.coli K-l2 LPS core. Furthermore, the most likely requirement in terms of core sugars is that a terminal di-glucose must be present since "galE" type LPS is an efficient acceptor of Vc OAg chains (Figure I).

The Salmonella LPS core has no terminal di-glucose because of the position of the galactose residue, and a Salmonella galE mutant produces LPS with a single terminal glucose (Figure I).

These observations support our hypothesis that the Salmonella LPS core is not a suitable acceptor for Vc OAg.

Accordingly, in a first aspect, the present invention provides an avirulent strain of Salmonella including a fragment of DNA containing genes encoding the synthesis of at least a portion of the core region of an E.coli strain.

The E.coli strain may be an E .coli K-l2 strain. The locus included in the fragment of DNA is referred to as rfa. Thus, to circumvent the problem of the prior art, Salmonella-E.coli K-l2 hybrid strains were constructed by transferring to Salmonella the region of the E.coli K-l2 chromosome which encodes the enzymes required to make the core region of the LPS. This locus, rfa, is located at about 8l min. on the E.coli K-l2 genetic map.

Such Salmonella -E.colihybrid strains, with rfa from E.coli K-l2 (rfa)$^{K-12}$, when constructed to harbour the appropriate plasmid clone, may express Vc OAg at high levels in the form of a lipopolysaccharide, and furthermore can also produce Salmonella O-somatic antigen in the form of a lipopolysaccharide.

In order to construct the Salmonella-E.coli hybrid strains described above, in a further aspect the present invention provides an E.coli strain modified to encode specific antibiotic resistance.

The E.coli strain may be an E.coli K-I2 strain. The E.coli K-I2 strain may be of any suitable type. An E.coli K-I2 strain E.coli K-I2 PK3 whose relevant characteristics are Hfr thr leu thi may be used. An E.coli K-I2 KL228 strain whose relevant characteristics are HFr thi leu may be used. It will be understood that the modification to encode for specific antibiotic resistance makes these strains suitable for isolation via simple selection techniques as described below.

For example, the E.coli strains may be encoded for resistance to chloramphenicol. The E.coli K-I2 strain may be modified to include an mtl:: Tn 9 insertion mutation. The modification may be undertaken by transduction.

In the description following reference will be made to a number of Escherichia coli, Salmonella typhimurium and Salmonella typhi strains by their codes. Each of these strains is maintained in the culture collection of the University of Adelaide, Australia. Table I below sets out the codes and the relevant characteristics of each of the bacterial strains referred to below.

## TABLE 1

### Bacterial Strains

| Escherichia coli K-12 strains | Relevant characteristics |
|---|---|
| PK3 | Hfr thr leu thi |
| KL228 | Hfr thi leu |
| NK6701 | mtl-16::Tn9 |
| EX178 | Hfr thi thyA [pPM1004] |
| EX170 | PK3 mtl-16::Tn9 |
| EX173 | KL228 mtl-16::Tn9 |
| P2495 | tolC210::Tn10 |

```
    EX260              EX170 tolC210::Tn10

    EX320              Hfr  thi  thyA  tolC210::Tn10 [pEVX14]*
```

Salmonella typhimurium strains

```
    G30                galE

    V490               G30 str^R [pPM1004]

    EX206              V490 (rfa^K-12, cml^R from E.coli K-12)

    EX262              G30 (rfa mtl-16::Tn9)^K-12

    LB5010             trp  metA  metE  ilv  leu  xyl  galE
                       hsdL  hsdS  hsdT  rpsL

    EX200              LB5010 (met^+, ilv^+, rfa,
                        mtl-16::Tn9)^K-12

    EX216              EX200 [pPM1004]

    EX361              EX200 thyA

    SGSC262            trp  metA  metE  ilv  xyl  hsdL  hsdS
                       rpsL  rfaG3037

    EX225              SGSC262 (ilv^+ xyl^+ rfa mtl-16::Tn9)^K-12
```

Salmonella typhi strains

```
    V487               Ty21a rif^R [pPM1004]

    EX210              V487 (rfa mtl-16::Tn9)^K-12

    EX233              EX210 cured of pPM1004

    EX256              EX233 mtl^+

    EX259              EX256 thyA

    EX363              EX259 [pEVX14]
```

* ( )^K-12, indicates genetic region from E.coli K-12

* pEVX14 is pEVX5 with a 20kb SacI fragment from pEVX3
  encoding the Ogawa-type O-antigen of Vibrio cholerae.
  (Described in Australian Patent Application 38900/85).

Examples of the E. coli K-l2 donor strains for use in the preparation of the hybrid strains described above are those designated EXI70, EXI73, EXI78, EX260 and EX320, samples of which are maintained in the culture collection of the University of Adelaide, Australia. Accordingly, in a further preferred aspect of the present invention there is provided E.coli donor strains EXI70, EXI73, EXI78, EX260 and EX320.

Examples of the Salmonella donor strains for use in the preparation of the hybrid strains described above are those designated Salmonella typhimurium V490 and Salmonella typhi V487, samples of which are maintained in the culture collection of the University of Adelaide, Australia. Accordingly, in a further preferred aspect of the present invention there is provided Salmonella donor strains Salmonella typhimurium V490 and Salmonella typhi V487.

The rfa locus of E.coli K-12 maps at about 8l min on the genetic map, very close to the genes for mannitol utilization, mtl. The (rfa)^K-12 region was introduced into Salmonella by means of Hfr mediated chromosomal gene transfer. The Hfr PK3 was used: the origin of transfer is at about 78 min, and transfer of (rfa)^K-12 as an early marker occurs in an anti-clockwise direction. In order to be able to select for the transfer of E.coli genes, strain PK3 was modified by transduction to have a mtl::Tn9 insertion mutation [Tn9 encodes resistance to chloramphenicol]. The mtl locus is located between the Hfr transfer original and (rfa)$^{K-12}$, and the chloramphenicol resistance determinant allows direct selection for transfer of the E.coli chromosome. Since mtl and (rfa)$^{K-12}$ are closely linked, it was expected that the majority of recipients receiving mtl::Tn9 would also receive (rfa)$^{K-12}$.

The donor strain constructed was called EXI70. It can be contraselected with an antibiotic to which the recipient is resistant. This donor also has several growth factor requirements (threonine and leucine requirement) which can also be used to contraselect the donor.

Alternatively, or in addition, the E.coli K-I2 donor strain may be modified to encode specific sensitivity to sodium deoxycholate. The E.coli may be modified to include a tolC2I0::TnI0 insertion mutation. The mutation may be inserted utilising a transduction.

For example, another donor strain, called EX260, is described. It is derived from EXI70 and differs from it in that it has a tolC2I0::TnI0 insertion mutation. This additional mutation allows the donor to be contraselected by using 0.02%w/v sodium deoxycholate in the selection media. This would allow selection for transfer of (rfa)$^{K-12}$ on nutrient medium since both chloramphenicol and deoxycholate are all that is needed to select Salmonella-E.coli hybrids.

Other Hfr donor strains may be constructed utilising similar techniques. Utilising a similar process to that used to construct EXI70, the E .coli strain KL228 which transfers the rfa region early in a clockwise direction (from an origin at about 84 min) was made mtl::Tn9 by transduction. This resulting strain was called EXI73.

The donor strains so formed may be utilised in the preparation of Salmonella-E.coli hybrids as described above. Thus, in a still further aspect of the present invention there is provided a method of preparing an avirulent Salmonella strain including a fragment of DNA containing genes encoding the synthesis of at least a portion of the core region of an E.coli strain which method includes providing

(i) an avirulent strain of Salmonella, and

(ii) an E.coli strain modified to encode specific antibiotic resistance; subjecting the strains to chromosomal gene transfer; and

selecting hybrid products so formed utilising the specific antibiotic resistance encoded in the E. coli strain.

The chromosomal gene transfer may be a Hfr mediated chromosomal gene transfer.

The E.coli strain may be selected from the donor strains described above.

In a preferred form, the avirulent strain of Salmonella may include a defined non-revertible mutation in the galE gene. The galE mutation may thus provide a test for the Salmonella-E.coli hybrids formed.

The avirulent Salmonella strains may be Salmonella typhi strains or Salmonella typhimurium strains. The Salmonella strains may be selected from Salmonella typhimurium strain LB50I0 Salmonella typhimurium LT2 rfaG , Salmonella typhimurium G30, variants thereof and mutants thereof.

In a preferred form, the avirulent strain of Salmonella may further include
a fragment of DNA containing genes encoding the synthesis of an O-antigen.
The O-antigen may be the Vibrio cholerae O-antigen (Vc OAg).
The avirulent strain of Salmonella may be modified to include the genes expressing the O-antigen via transformation with a suitable plasmid.

Thus, in a preferred aspect, the method of preparing an avirulent Salmonella strain as described above may further include
providing a plasmid containing genes expressing an
O-antigen and bearing a thyA$^+$ non-antibiotic marker, and
a thyA derivative of the avirulent Salmonella strain described above; and
inserting the cloned genes on the plasmid into the chromosome of the avirulent thyA Salmonella strain.

Techniques for preparing plasmids bearing thyA$^+$ non-antibiotic markers are described in our co-pending Australian Patent Application 74202/87 the entire disclosure of which is incorporated herein by reference. Plasmids pEVX8 and pEVX9 described therein as expressing the O-antigen and having thyA$^+$ as a positive selection marker may be used.

Examples of avirulent Salmonella-E.coli hybrid strains of the type described above are those designated EDX200, EX225, EX262, EX206, EX2I0 and EX363 described below. Samples of each of these strains are maintained in the culture collection of the University of Adelaide, Australia.

Accordingly, in a preferred aspect of the present invention, there is provided the avirulent Salmonella-E. coli hybrid strains
EX200    LB50I0 (met$^+$, ilv$^+$, rfa, mtl-I6::Tn9)> $^{K-12}$from E.coli$^{K-12}$
EX225    SGSC262 ilv$^+$ xyl$^+$ rfa mtl-I6::Tn9)$^{K-12}$
EX262    G30 (rfa mtl-I6::Tn9 )$^{K-12}$

EX206   V490 (rfa$^{K-12}$, cml$^R$ from E.coli K-l2)
EX2l0   V487 (rfa mtl-l6::Tn9)$^{K-12}$
EX233   EX2l0 cured of pPMl004
EX256   EX233 mtl$^+$
EX259   EX256 thyA
EX363   EX259 [pEVXl4]

According to a still further aspect of the present invention, there is provided a vaccine composition useful for effecting immunity against enteric diseases including an avirulent strain of Salmonella including a fragment of DNA containing genes encoding the synthesis of at least a portion of an E.coli strain; and a fragment of DNA containing genes encoding the synthesis of an O-antigen.

The enteric disease may be cholera. The O-antigen may be Vibrio cholerae O-somatic antigen (Vc OAg).

The avirulent strain may be selected from EX2l0 and EX363.

In a further aspect of the present invention, there is provided a method for the prophylactic treatment of enteric diseases in mammals which method includes administering to the mammal a prophylactically effective amount of the vaccine described above.

The invention will now be more fully described with reference to the accompanying examples. It should be understood, however, that the description following is illustrative only and should not be taken in any way as a restriction on the generality of the invention described above.

## Construction of strain EX200

Strain EXl70 was mated with the Salmonellatyphimurium strain LB50l0. Exconjugants which were str$^R$ - (streptomycin resistant), ilv$^+$ met$^+$ (growth without isoleucine and valine, and methionine), and chloramphenicol resistant (mtl::Tn9) were selected.

Such Salmonella-E.coli hybrids were expected to have E.coliK-l2 chromosome between 80 min and 88 min, replacing the equivalent region of the Salmonella chromosome. Such exconjugants must have the (rfa)-$^{K-12}$ locus. Since strain LB50l0 is also a galE mutant, the effect on the LPS of Salmonella typhimurium could be determined by testing with LPS-dependent bacteriophages. Phage P22 requires O-antigen as its receptor, phage Felix O (FO) requires intact core, and phages Pl and C2l require a core with terminal glucose.

Salmonella-E.coli hybrids of the desired phenotype were found among the exconjugants. One such hybrid, called EX200 was found to be resistant to phage FO when tested on media containing galactose. FO is a phage which uses intact Salmonella LPS core region to adsorb and does ot infect E.coli K-l2. Hence the Salmonella rfa locus most likely has been replaced by the E.coli K-l2 rfa locus. Since EX200 is sensitive to phage P22 when the growth media contains galactose, this demonstrates that the Salmonella typhimurium O-antigen is assembled onto the E.coli K-l2 LPS core sugars. These observations are supported by the data obtained with Salmonella typhi (see below).

To test if a Salmonella strain with (rfa)$^{K-12}$could express the Vc OAg, plasmids pEVX8 and pEVX9 [these plasmids are described in Australian patent application number      ] were transformed into a thyA derivative of EX200, called EX36l. Such transformants expressed the Vc OAg as judged by slide agglutination with an anti-Vibrio cholerae serum.

In another experiment, plasmid pPMl004 was introduced into EX200 by mating with EXl78. One exconjugant strain EX2l6, produced Vc OAg comparable to E,coliK-l2 strains having pPMl004, as judged by silver staining of celll envelopes electrophoresed on an SDS-polyacrylamide gel.

## Construction of strain EX225

Strain EXl70 was mated with SGSC262, a Salmonella typhimurium LT2 rfaG mutant, and cml$^R$ - (chloramphenicol resistant), str$^R$exconjugants were selected. All exconjugants tested become rfaG$^+$, namely they become sensitive to the O-antigen specific phage P22, and resistant to the core-specific phage Pl and C2l. Hence the rfa region of E.coli K-l2 can be efficiently transferred to Salmonella, confirming the results above. An example of such an exconjugants is EX225. This strain and all other exconjugants obtained were ilv$^+$ (located at 84 min.), indicating that this marker is closely linked to mtl and rfa. Other exconjugants also became xyl$^+$ (located at 79 min.), but none were met$^+$ (located at 89 min.). Hence the amount of E.coli chromosome received from the donor is most limited to a few minutes on either side of the mtl marker.

## Construction of Strain EX262

A derivative of Salmonella typhimurium G-30 which had rfa)$^{K-12}$ region was constructed as follows.

The generalised transducing phage P1vir was grown on strain EX200. This phage lysate was used to infect G30 and chloramphenicol resistant transductant colonies were selected. One transductant was recovered, called EX262, which was mtl⁻ (i.e. mtl::Tn9) and resistant to phage FO on nutrient medium with galactose. Most transductants were mtl⁺ suggesting that the transposon Tn9 had moved to another location during the transduction event.

Strain EX262 behaves identically to G30 in terms of its ability to colonize Peyer's patches in mice. This indicates that the (rfa)$^{K-12}$ did not influence this characteristic of Salmonella typhimurium. It should be noted that few transductants were obtained in this cross. This most likely is due to a greatly reduced recombination frequency caused by the E.coli K-l2 chromosomal DNA on either side of (mtl::Tn9)$^{K-12}$ in strain EX200.

## Construction of strain EXI06

Strain V490 is Salmonella typhimurium G30 but str$^R$ and carries pPMI004 (tet$^R$, tetracycline resistant, Vc OAg clone). This strain expresses Vc OAg poorly. Strain EXI73 was mated with V490 and cml$^R$, str$^R$, tet$^R$ - (pPMI004 retention), exconjugants were selected.

Among the exconjugants, which were screened for Vc OAg production by SDA-PAGE and silver staining, one was found which expressed high levels of Vc OAg. This strain was called EX206.

Strain EX206 was found to be mtl⁺ (i.e. manitol fermention positive), yet was cml$^R$. This strain may have Tn9 relocated to another place in the chromosome (see above). The alternative possibility is that an F'mtl::Tn9 was transferred during the mating. However, examination of the plasmid content of EX206 did not reveal the presence of and F' but it does contain pPMI004 and the Salmonella typhimurium cryptic plasmid.

## Construction of strain EX2l0

Strain V487, which is Ty2la but rif$^R$ (rifampicin resistant) and harbours pPMI004 (tet$^R$, Vc OAg cone), was mated with strain EXI70 and cml$^R$ rif$^R$ tet$^R$ exconjugants were selected on the appropriately supplemented nutrient media. The resulting colonies were examined for Vc OAg expression by silver staining of cell envelope material separated on an SDS-polyacrylamide gel. The presence of LPS molecules with the E.coli K-l2 core instead of Salmonella core in the hybrid strains could be easily detected by silver staining of cell envelopes or whole cell lysates separated by SDS-PAGE. LPS with E.coli core migrates relatively slower than LPS with Salmonella core. Furthermore, the mobility of the LPS in the hybrids resemble that in E.coli K-l2. One exconjugant which expressed Vc OAg and was mtl⁻ was called EX2l0.

Strain Ex2l0 was resistant to phage FO even when galactose was added to media, but sensitive to phage P22 indicating that the Salmonella O-antigen is expressed in this strain. This strain produces Vc OAg as LPS since it is extractable by the hot phenol/water method. Vc OAg is detectable by silver staining of cell envelopes, or whole cell lysates separated by SDS-PAGE. When galactose is added to the media, both Salmonella O-antigen and Vc OAg can be detected by silver staining. EX2l0 cells can be agglutinated by antiserum to Vibrio cholerae showing that the OAg is on the cell surface. isolated LPS from EX2l0 inhibits specifically in a haemagglutination inhibition assay, using SRBC coated with Vibrio cholerae 569B LPS. Formalin fixed EX2l0 cells also inhibit in such an assay. These assays suggest that EX2l0 produces l0%-50% of the Vc OAg produced by Vibrio cholerae itself on a weight basis.

LPS isolated from EX2l0, EX2l0 killed by formalin, and live EX2l0 itself can elicit an immune response to 569B LPS in rabbits.

Strain EX2l0 is a potential live oral vaccine for cholera.

## Construction of Strain EX363

Strain EX2I0 was cured of pPMI004 by spontaneous loss to give EX233. A spontaneous $mtl^+$ revertant of EX233 was selected on minimal media and was called strain EX256. A thyA mutant of EX256 was selected by plating on minimal media with thymine and trimethoprim. This strain was called EX259 and was found to be non-reverting ($10^{-8}$). Hence EX259 is Ty2la $rif^R$ (rfa) $^{K-12}$ thyA. The$thyA^+$ Vc OAg (Ogawa type) plasmid pEVXI4 was transferred to strain EX259 by mating strain EX259 with strain EX320. Selection was for $thy^+$ $DOC^R$ (deoxycholate resistant) exconjugants. the resulting colonies were screened for plasmid content. One exconjugant was found which had pEVXI4, and was called strain EX363. This strain expressed Vc OAg as judged by silver staining of whole cell lysates treated with proteinase K and electrophoresed on SDS-polyacrylamide gel.

EX363 is a potential live oral vaccine for cholera, and has only one antibiotic resistant marker, $rif^R$.

The Salmonella (rfa )$^{K-12}$ hybrid strains may be useful for the expression of O-antigens from other gram-negative pathogens, if such O-antigens are protective antigens: an example of this may be the O-antigen of Shigella species. Further modification of the Salmonella strains may be required if expression is not achieved. For example, expression may require (rfa)$^{K-12}$ and another chromosomal region of E.coli K-I2.

Apart from the E.coli K-I2 LPS core sugar structure (Figure I), at least four other types are known. Such core types may be required for the expression of various types of O-antigen. Hence a series of Salmonella strains could be constructed with each of LPS core types. These would be useful for the expression of O-antigens from different gram-negative pathogen.

## METHODS

### Bacterial Strains and Growth Media

The bacterial strains used, and their relevant characteristics are listed in Table I.
Bacterial strains were grown in one of the following media:
    (i) LB:      I0g/l Bacto-Tryptone
5g/l Bacto-Yeast Extract
5g/l NaCl
    (ii) Difto NB      I6g/l Difco Nutrient Broth
5g/l NaCl
    (iii) Oxoid NB      I0g/l Oxoid Lab Lemco
I0g/l Oxoid Bacteriological Peptone
5g/l NaCl
Galactose was added to media at 0.00I% w/v when expression of Salmonella O-antigen, in galE mutants, was required.
Oxoid agar was added at I.5% w/v to solidify media.

### Bacteriophage Sensitivity Testing

Bacterial cultures, grown in Difco NB, with and without galactose added, were streaked with cotton swabs on the same media. The phage to be tested was spotted (2.5 ul) onto the dried streak. After incubation at 37°C for I6h, the sensitivity to the phage was scored.

### Hfr matings

Bacterial cultures were grown in LB or Difco NB for I6h. The donor (0.5 ml) and recipient (5 ml) were mixed, concentration by centrifugation and plated onto a nitrocellulose filter placed on the surface of a nutrient agar plate (LB agar, or Difco NB agar). Matings were incubated at 37°C for 5-6h. The cells were resuspended in broth and plated on selection media.

## PI transduction

Transduction with PIvir were performed as described by Miller (1972). In order to use PIvir in Salmonella, a stock of PIvir was first prepared on strain LB50l0.

## Analysis of LPS

(i) Cell envelopes were prepared by treatment of cells with lysozyme, EDTA, followed by sonication and high speed centrifugation to recover all the insoluble material.

(ii) Protinase K treatment of whole cell lysates was performed as described by Hitchcock and Brown (1983). The samples were examined by SDS/PAGE.

(iii) LPS was extracted from I to 6 titres of bacterial culture by the hot phenol/water method of Westphal and Jann (1965).

(iv) Isolated LPS or formalin fixed cells were tested by an HIA haemagglutination inhibition assay using SRBC coated with Inaba LPS, or Ogawa LPS, or Salmonella LPS, and the appropriate antiserum.

## SDS/PAGE and silver staining

SDS/PAGE was performed on 20% polyacrylamide gels as described by Manning et al (1986).

Gells were stained for LPS as described by Tsai and Fratsch (1982) or Hitchcock and Brown (1983).

Finally, it is to be understood that various other modifications and/or alterations may be made without departing from the spirit of the present invention as outlined herein.

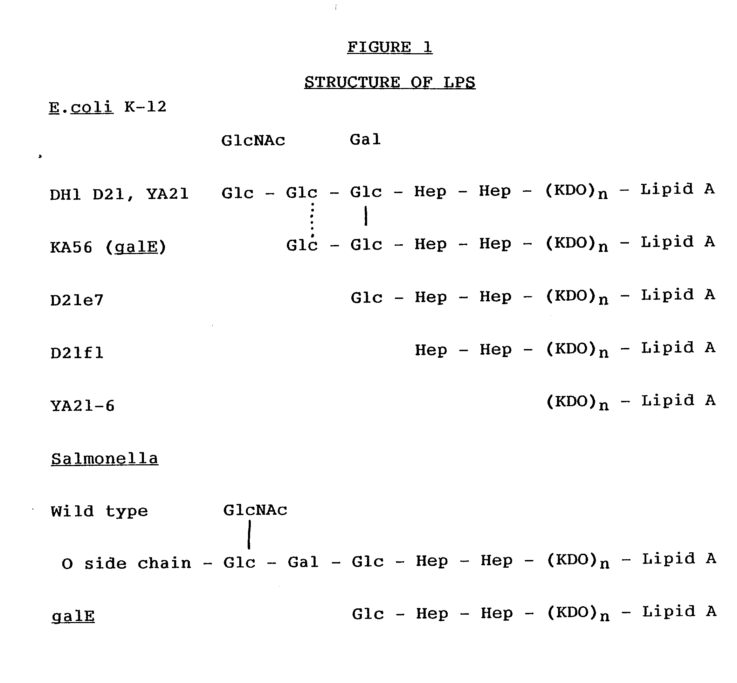

## Claims

1. An avirulent strain of Salmonella including a fragment of DNA containing genes encoding the synthesis of at least a portion of the core region of an E. coli strain.

2. An avirulent strain according to claim I wherein the Salmonella strain is a Salmonella typhimurium or Salmonella typhi strain.

3. An avirulent strain according to claim 2 wherein the Salmonella typhimurium or Salmonella typhi strain are selected from Salmonella typhimurium strain LB50l0, Salmonella typhimurium LT2 rfaG, Salmonella typhimurium G30, Salmonella typhi V487, variants thereof and mutants thereof.

4. An avirulent strain according to claim I wherein the E.coli strain is an E.coli K-l2 strain and the fragment of DNA includes the locus, rfa, located at approximately 8l min on the E.coli K-l2 genetic map and including the enzymes required to form the core region of the lipopolysaccharide.

5. An avirulent strain according to claim 2 wherein the E.coli strain is modified to encode specific antibiotic resistance.

6. An avirulent strain according to claim 5 wherein the E.coli strain is encoded for resistance to chloramphenicol or deoxycholate.

7. An avirulent strain according to claim 4 wherein the E.coli K-l2 strain is selected from E.coli PK3, E.coli KL228, E.coliNK670l, E.coli EXl78, E.coli EXl70, E.coli EXl73, E.coli P2495, E.coli EX260, E.coli EX320, as hereinbefore described; variants thereof and mutants thereof.

8. An E.coli donor strain selected from EXl70, EXl73, EXl78, EX260 and EX320 as hereinbefore described; variants thereof and mutants thereof.

9. A Salmonella donor strain selected from Salmonellatyphimurium V490 and Salmonella typhi V487, as hereinbefore described.

l0. An avirulent Salmonella-E.coli hybrid strain selected from EX200, EX225, EX262, EX206, EX2l0, EX233, EX256, EX259, EX363, as hereinbefore described; variants thereof and mutants thereof.

Il. A method of preparing an avirulent strain of Salmonella including a fragment of DNA containing genes encoding the synthesis of at least a portion of the core region of an E.coli strain which method includes providing

(i) an avirulent strain of Salmonella, and

(ii) an E.coli strain modified to encode specific antibiotic resistance;

subjecting the strains to chromosomal gene transfer; and

selecting hybrid products so formed utilizing the specific antibiotic resistance encoded in the E.coli strain.

The E.coli strain may be the donor strains described above.

12. A method according to claim II wherein the chromosomal gene transfer is a Hfr mediated chromosomal gene transfer.

13. A method according to claim I2 wherein the E. coli strain is selected from EXI78, EXI70, EXI73, EX260 and EX320.

14. A method according to claim I3 wherein the avirulent strain of Salmonella includes a defined non-revertible mutation of the galE gene.

15. A method according to claim I4 wherein the Salmonella strain is a Salmonella typhi or Salmonella typhimurium strain.

16. A method according to claim I5 wherein the Salmonella strain is selected from Salmonella typhimurium strain LB50I0, Salmonella typhimurium LT2 rfaG, Salmonella typhimurium G30, variants thereof and mutants thereof.

17. A method according to claim I6 wherein the avirulent strain of Salmonella further includes

(c) a fragment of DNA containing genes encoding the synthesis of an O-antigen.

18. A method according to claim I7 wherein the O-antigen is the vibrio cholerae O-antigen (Vc OAg).

19. A method according to claim I7 further including providing a plasmid containing genes expressing an O-antigen and bearing a thyA$^+$ non-antibiotic marker, and

a thyA derivative of the avirulent Salmonella strain described above; and

inserting the cloned genes on the plasmid into the chromosome of the avirulent thyA Salmonella strain.

20. A method according to claim I9 wherein the plasmids are selected from plasmids pEVX8 and pEVX9, as hereinbefore described.

21. A vaccine composition useful for effecting immunity against enteric diseases including an avirulent strain of Salmonella including a fragment of DNA containing genes encoding the synthesis of at least a portion of an E. coli strain; and a fragment of DNA containing genes encoding the synthesis of an O-antigen.

22. A vaccine composition according to claim 2I wherein the enteric disease is cholera.

23. A vaccine composition according to claim 22 wherein the O-antigen is the Vibrio cholerae O-somatic antigen (Vc Og).

24. A vaccine composition according to claim 2I wherein the avirulent strain of Salmonella is selected from EX200, EX225, EX262, EX206, EX210, EX233, EX256, EX259, EX363, as hereinbefore described; variants thereof and mutants thereof.

25. A method for the prophylactic treatment of enteric diseases in mammals which method includes administering to the mammal a prophylactically effective amount of a vaccine composition useful for effecting immunity against enteric diseases including an avirulent strain of Salmonella including a fragment of DNA containing genes encoding the synthesis of at least a portion of an E .coli strain; and a fragment of DNA containing genes encoding the synthesis of an O-antigen.

0 257 837

## FIGURE 1

### STRUCTURE OF LPS

E.coli K-12

```
                   GlcNAc           Gal

DH1 D21, YA21   Glc - Glc - Glc - Hep - Hep - (KDO)n - Lipid A
                            :           |
                            :
KA56 (galE)           Glc - Glc - Hep - Hep - (KDO)n - Lipid A


D21e7                       Glc - Hep - Hep - (KDO)n - Lipid A


D21f1                             Hep - Hep - (KDO)n - Lipid A


YA21-6                                        (KDO)n - Lipid A
```

Salmonella

```
Wild type          GlcNAc
                     |
  O side chain - Glc - Gal - Glc - Hep - Hep - (KDO)n - Lipid A


galE                              Glc - Hep - Hep - (KDO)n - Lipid A
```

European Patent Office

**PARTIAL EUROPEAN SEARCH REPORT**

which under Rule 45 of the European Patent Convention shall be considered, for the purposes of subsequent proceedings, as the European search report

Application number

EP 87 30 6833

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,O | BIOLOGICAL ABSTRACTS/RMM, vol. 30, ref.no. 116588, Philadelphia, Penn. US; P.R. MACLACHLAN et al.: "Cloning of the RFA-KL genes for lipopolysaccharide synthesis in Salmonella-typhimurium", & ABSTRACTS OF THE ANNUAL MEETING OF THE AMERICAN SOCIETY FOR MICROBIOLOGY(US) 1986, vol. 86, no. 9 | | C 12 N 15/00<br>A 61 K 39/108<br>A 61 K 39/112<br>A 61 K 39/106 |
| | * Title, terms and biosystematic code * | 1-10 | |
| Y | | 11-24 | |
| | -- | | |
| X | CHEMICAL ABSTRACTS, vol. 91, no. 4, 23rd July 1979, page 298, ref.no. 35502w, Columbus, Ohio, US; E.S. CREEGER et al.: "Cloning of genes for bacterial glycosyl-transferases. I. Selection of hybrid plasmids carrying genes for two glycosyltransferases", & J.BIOL.CHEM. 1979, 254(3), 804-10 | | **TECHNICAL FIELDS SEARCHED (Int. Cl.4)** |
| | * Abstract * | 1-10 | C 12 N<br>A 61 K |

## INCOMPLETE SEARCH    -2-

The Search Division considers that the present European patent application does not comply with the provisions of the European Patent Convention to such an extent that it is not possible to carry out a meaningful search into the state of the art on the basis of some of the claims.

Claims searched completely: 1-24

Claims searched incompletely:

Claims not searched: 25

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 02-12-1987 | SKELLY |

EPO Form 1505.1 03.82

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | |
| Y | -- | 11-24 | |
| Y | BIOLOGICAL ABSTRACTS, vol. 64, 1st November 1977, page 4917, ref.no. 50115, Philadelphia, Penn. US; C. GODARD et al.: "Study of an rfa locus coding for cell wall lipopolysaccharide core biosynthesis in Shigella flexneri F6S", & ANN.MICROBIOL. (PARIS) 128A(1): 19-34,1977 | | |
| | * Abstract * | 11-20 | |
| D,Y | -- INFECTION AND IMMUNITY, vol. 53, no. 2, August 1986, pages 272-277, American Soc. for Microbiology, US; P.A. MANNING et al.: "Molecular cloning and expresion in Escherichia coli K-12 of the O antigens of the Inaba and Ogawa serotypes of the Vibrio cholerae O1 lipopoly- saccharides and their potential for vaccine development" | | TECHNICAL FIELDS SEARCHED (Int. Cl.4) |
| | * Whole document * | 17-24 | |
| D,Y | -- WO - A - 85 03 521 (ENTEROVAX RESEARCH PTY) | | |
| | * Example 5; claims 14-27,29,31 * | 17-24 | |
| A | -- US - A - 3 856 935 (GERMANIER) | | |
| | * Whole document * | 1 | |
| A | -- CHEMICAL ABSTRACTS, vol. 79, no. 15, 15th October 1973, page 198, ref.no. 89374b, Columbus, Ohio, US; G. SCHMIDT: "Genetical studies on the lipopolysaccharide structure of Escherichia coli K12", & J.GEN.MICROBIOL. 1973, 77(Pt.1) 151-60 | | |
| | * Abstract * | 1 | |
| A | -- CHEMICAL ABSTRACTS, vol. 102, no. 17, 29th April 1985, page 318, ref.no. 145976g, Columbus, Ohio, US; W.G. COLEMAN Jr et al.: "New cysE-pyrE-linked rfa mutation in Escherichia coli K-12 that results | | |

-3-

| Category | Citation of document with indication, where appropriate, of relevant passages | Relevant to claim |
|---|---|---|
| | in a heptoseless lipopolysaccharide" & J.BACTERIOL. 1985, 161(3),1209-14 * Abstract * | 1 |

**DOCUMENTS CONSIDERED TO BE RELEVANT**

CLASSIFICATION OF THE APPLICATION (Int. Cl.4)

TECHNICAL FIELDS SEARCHED (Int. Cl.4)